# EUROPEAN PATENT APPLICATION

(11) **EP 2 689 711 A2**
(43) Date of publication of application: **29.01.2014**
(21) Application number: 13157520.1
(22) Date of filing: 02.03.2013
(51) Int. Cl.: A61B 1/00

(54) **Pipe endoscope**

(30) Priority: 03.07.2012 JP 2012149793
(71) Applicant: Coden Co., Ltd., Tokyo 114-0024 (JP)
(72) Inventor: Koda, Yoshiharu, Tokyo, 114-0024 (JP); Koda, Kojiro, Tokyo, 114-0024 (JP)
(74) Representative: McCartney, Jonathan William

(57) **Abstract**

A pipe endoscope includes: a transparent observation container having a bottomed cylindrical shape which is open on a base end side and sealed on a tip side; a scope which is inserted into the observation container and includes an objective lens at a tip of the scope; and an image processing device which is connected to the scope and processes information collected by the scope to display a video image. The observation container includes: a protrusion which is projected to the tip side beyond a sealed bottom part having light-transmitting property, and a tip of which is processed to have curvature; and a detachable light source which irradiates the periphery of the bottom part with light.

## Description

### BACKGROUND

### 1. Technical Field

The present invention relates to a pipe endoscope which visually examines an affected part in a patient's body by a video image.

### 2. Description of Related Arts

An endoscope is a medical instrument which visually observes an affected part by a video image by inserting an insertion part of the endoscope including an optical system into a patient's body.

The insertion part of the endoscope is directly exposed to the internal environment of the patient's body when performing an examination using the endoscope. In order to prevent nosocomial infection, the entire instrument thus always needs to be sterilized to a sterile condition after directly observing the affected part in the patient's body using the endoscope. The sterilization of the endoscope however requires time and a cost. In particular, when an endoscope including a large-sized camera is used, sterilization equipment also needs to be increased in scale, thereby having caused the maintenance cost of the sterilization equipment to run up.

Accordingly, in recent years, various technologies have been proposed in which the insertion part of the endoscope does not come in direct contact with inside body of the patient. As a technology to prevent contamination of the endoscope, a contamination prevention device for the endoscope has been disclosed, the contamination prevention device including a cylindrical cover which detachably covers and shields the insertion part of the endoscope from the outside (refer to Japanese Laid-Open Patent Publication No. 2003-126011, for example).

According to Japanese Laid-Open Patent Publication No. 2003-126011, the cylindrical cover is a cylindrical body having a closed shape except for an opening at the base end, is formed of an elastic material, and has an inner diameter larger than an outer diameter of the insertion part. Also provided is cover fixing means which fixes the cylindrical cover to the endoscope by stretching the cylindrical cover out to the base end side and reducing the diameter thereof so that the cylindrical cover becomes tightly fixed to the entire surface of the insertion part being inserted in the cylindrical cover. The cover fixing means can be released freely.

The endoscope in the related art is inserted from an esophagus and a respiratory tract to observe and treat the affected part, whereas the endoscope described herein is inserted directly from a body surface to the affected part for observation.

The contamination prevention device of the endoscope in Japanese Laid-Open Patent Publication No. 2003-126011 uses the elastic cylindrical cover to cover the insertion part of the endoscope, the cylindrical cover having the closed shape except for the opening at the base end. Since the insertion part of the endoscope is covered by the cylindrical cover, the amount of light enough to observe the affected part in the patient's body is not always obtained.

Moreover, the tip of the cylindrical cover being a closed plane surface, it is concerned that the view of the endoscope would be obstructed when the tip of the cylindrical cover is contaminated with body fluid or the like. It is also concerned that the plane surface at the tip of the cylindrical cover would interfere with an internal organ or the like to hinder smooth insertion of the endoscope into the patient's body.

The cylindrical cover in Japanese Laid-Open Patent Publication No. 2003-126011 is provided with a manually-operated screw as fixing means that can be released. The sterilization is not required for the endoscope but is required for the cylindrical cover which is not presupposed to be disposable.

The present invention has been invented in consideration of the aforementioned circumstances. An obj ect of the present invention is to provide a pipe endoscope in which a scope does not come in direct contact with the environment of the affected part in the patient's body, no sterilization is required, enough amount of light is provided for collecting information on the affected part, and the insertion operation can be performed smoothly.

### SUMMARY

The pipe endoscope for achieving the aforementioned object includes an observation container, a scope including an image fiberscope and a lens, and an image processing device such as a camera.

The observation container is a transparent container having a bottomed cylindrical shape which is open on the base end side thereof and sealed on the tip side.

The scope has an objective lens at the tip and is inserted into the observation container.

The image processing device is connected to the scope including the image fiberscope and the lens, and processes collected information to display a video image.

The observation container includes: a protrusion which is projected toward the tip side beyond a sealed bottom part having light-transmitting property, and a tip of which is processed to have curvature; and a detachable light source which irradiates the periphery of the bottom part with light.

According to the pipe endoscope of the present invention, the scope which has the objective lens at the tip and includes the image fiberscope and the lens is inserted in the transparent observation container, the bottom part of which is sealed, and is mounted with the image processing device such as the camera. That is, the scope is directly introduced into the affected part in the patient's body from outside body via the observation container.

At this time, the scope obtains enough amount of light to collect information on the affected part in the patient's body since the light from the light source is radiated from the bottom part of the observation container.

The light source is detachably provided to the observation container, so that the observation container can be disposed by detaching the light source.

Therefore, by having the disposable observation container, the pipe endoscope can be realized in which the scope does not come in direct contact with the environment of the affected part in the patient's body and no sterilization is required.

Moreover, the bottom part of the observation container would not be contaminated with the internal environment of the body owing to the protrusion provided toward the tip side beyond the bottom part, whereby satisfactory light-transmitting property can be maintained. Furthermore, since the tip of the protrusion has been processed to have the curvature R, the endoscope can be smoothly inserted into the patient's body and, at the same time, the internal organ or the like of the patient would not be harmed.

### BRIEF DESCRIPTION OF THE DRAWINGS

FIG. 1 is a schematic block diagram of a pipe endoscope according to the present embodiment;
FIGS. 2A and 2B are a cross-sectional view and a perspective view, respectively, of a direct-viewing observation container ( box or case ) of the present embodiment;
FIGS. 3A and 3B are a cross-sectional view and a perspective view, respectively, of a side-viewing observation container of the present embodiment;
FIG. 4 is a schematic block diagram of a light source and a light source supporting member of the present embodiment;
FIG. 5 is a schematic perspective view of a mount packing of the present embodiment;
FIG. 6 is a schematic perspective view of a scope and an image processing device of the present embodiment;
FIG. 7 is a schematic perspective view of a boring scalpel of the present embodiment;
FIG. 8 is a schematic diagram of a use example of the direct-viewing observation container in the pipe endoscope according to the present embodiment; and
FIG. 9 is a schematic diagram of a use example of the side-viewing observation container in the pipe endoscope according to the present embodiment.

### DETAILED DESCRIPTION

A pipe endoscope according to the present embodiment will now be described with reference to the drawings.

In the pipe endoscope according to the present embodiment, a scope having an objective lens at a tip is introduced into an affected part in a patient's body via a transparent observation container having a bottomed cylindrical shape which is open on a base end side and sealed on a tip side. Therefore, by making the observation container disposable, the pipe endoscope can be provided in which the scope and an image capturing unit do not come in direct contact with the environment of the affected part in the patient's body and no sterilization is required.

### < Configuration of pipe endoscope >

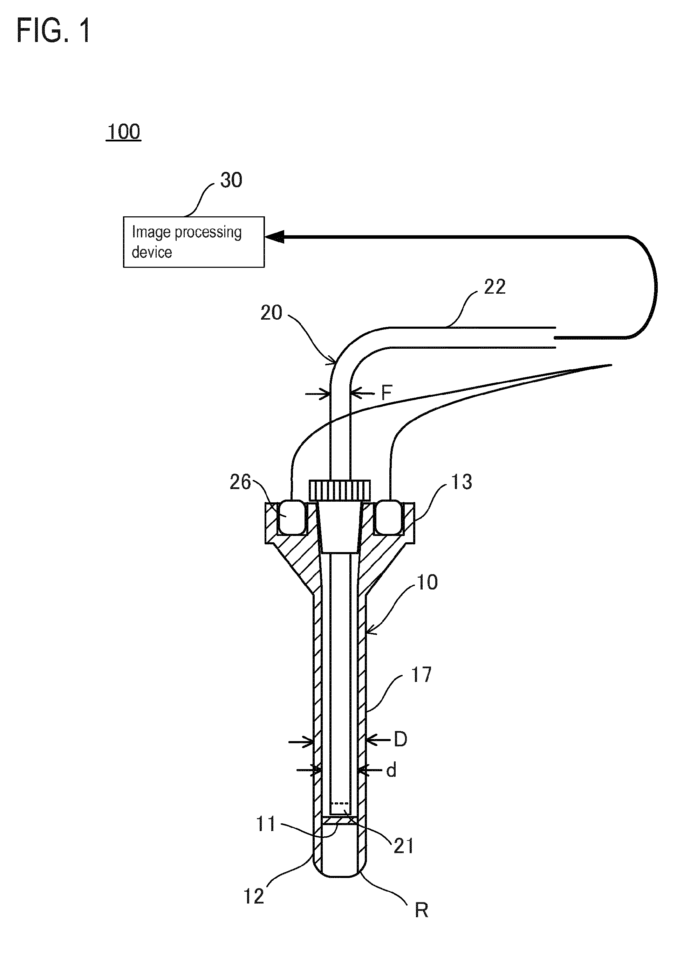

The configuration of the pipe endoscope according to the present embodiment will be described with reference to FIGS. 1 to 6.

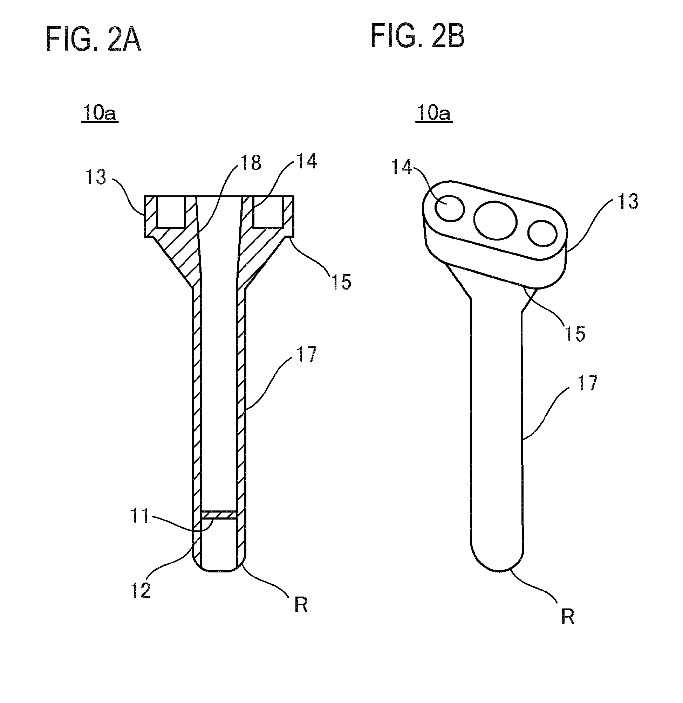

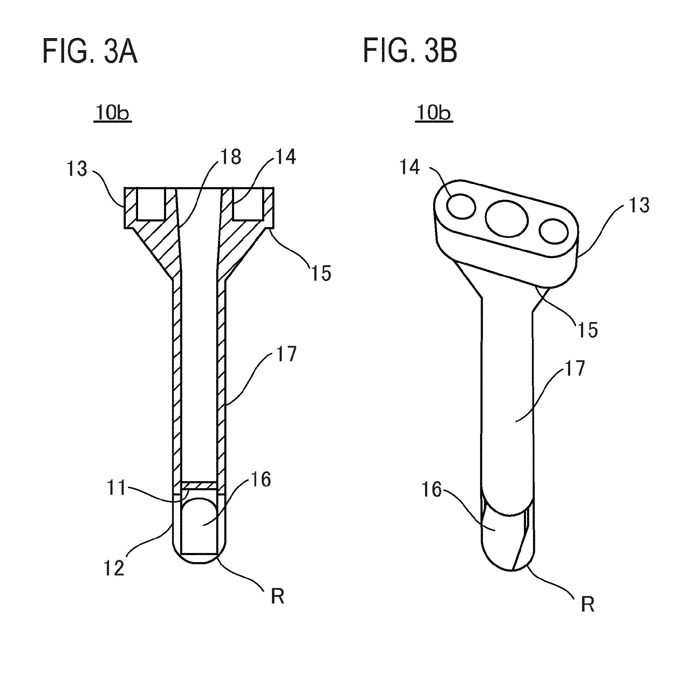

FIG. 1 is a schematic block diagram of the pipe endoscope according to the present embodiment. FIGS. 2A and 2B are a cross-sectional view and a perspective view, respectively, of a direct-viewing observation container in the present embodiment. FIGS. 3A and 3B are a cross-sectional view and a perspective view, respectively, of a side-viewing observation container in the present embodiment.

As illustrated in FIG. 1, a pipe endoscope 100 according to the present embodiment includes an observation container 10, a scope 20, and an image processing device 30.

As illustrated in FIGS. 1 to 3, the observation container 10 is formed of a container having the bottomed cylindrical shape which is open on the base end side and sealed on the tip side. The observation container 10 has a bottom part 11 in the vicinity of the tip of the observation container 10. The scope 20 having an objective lens 21 at the tip is inserted and fitted into the observation container 10. Theobservation container 10 is thus formed of a transparent material so that a user can visually recognize the exterior through the bottom part 11.

The interior of the observation container is formed of a material that would not be soaked by the internal environment of a body such as body fluid and that is harmless to a human body because the observation container 10 is directly exposed to the environment of a tissue of the affected part in the patient's body. The observation container 10 according to the present embodiment is formed of a synthetic resin that is colorless and transparent, for example. The length and the outer diameter of a cylindrical body 17 of the observation container 10 is set as appropriate according to the usage of the endoscope. The cylindrical body 17 would have flexibility by forming the observation container 10 of a flexible synthetic resin.

The bottom part 11 of the observation container 10 is formed of a plane surface. It is preferred that the bottom part 11 of the observation container 10 has superior light-transmitting property by, for example, forming the bottom part 11 thinner than the other part of the observation container 10.

The tip side of the observation container 10, namely the side of the bottom part 11, is inserted into the affected part in the patient's body. Therefore, a short-piped protrusion 12 is provided on the tip side beyond the bottom part 11 so that the bottom part 11 of the observation container 10 would not be contaminated with the internal environment of a body. The tip of the protrusion 12 is processed to have curvature R so as to insert the endoscope into the patient's body easily and not harm the internal organ or the like of the patient.

The scope 20 is inserted into the observation container 10. An inner diameter d of the observation container 10 is thus set larger than an outer diameter F of the scope 20. A gap is formed between the inner wall of the observation container 10 and the outer wall of the scope 20.

An enlarged-diameter part 13 is formed at the base of the observation container 10. The enlarged-diameter part 13 of the observation container 10 has a cross section in the shape of an arrow and has a thickness that is gradually increased toward the base end part. The top surface of the enlarged-diameter part 13 of the observation container 10 is formed into an oval, an elliptical, or a circular shape, for example.

The outer wall of the enlarged-diameter part 13 functions as a stopper for preventing the entire observation container 10 from entering the patient's body and as a grip for handling the observation container 10. On the other hand, the inner wall of the enlarged-diameter part 13 has a diameter that is gradually increased toward the base end part and thus functions as a guide for guiding the tip of the scope 20 into the observation container 10.

A light source housing part 14 which sets a light source 26 is opened on the top surface of the enlarged-diameter part 13. The light source housing part 14 is formed as a columnar hollow part. As illustrated in FIGS. 2B and 3B, the light source housing part 14 is distributed into two places in a longer direction of the top surface of the enlarged-diameter part 13 which is in the oval shape in the observation container 10 according to the present embodiment. When the top surface of the enlarged-diameter part 13 is in the circular shape, the light source housing part 14 may be distributed into four places, for example. The number of units of the light source housing part 14 is not limited.

Detachably housed inside the light source housing part 14 is the light source 26 by which light is radiated from the periphery of the bottompart 11 of the observation container 10. An LED lamp which is compact and has small power consumption can be used as the light source 26, for example.

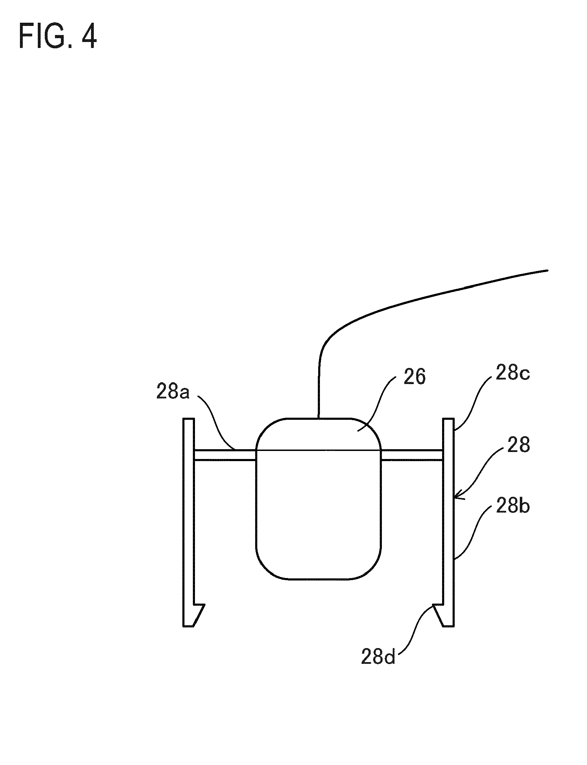

The light source 26 is detachably housed in the light source housing part 14 of the observation container 10. FIG. 4 is a schematic block diagram of the light source and a light source supporting member in the present embodiment.

As illustrated in FIG. 4, the light source 26 is housed in the light source housing part 14 of the observation container 10 as well as supported by a light source supporting member 28 at the bottom surface of the enlarged-diameter part 13 of the observation container 10. A supporting step 15 is formed at the bottom surface of the enlarged-diameter part 13 of the observation container 10.

The light source supporting member 28 is a substantially H-shaped member formed of a synthetic resin, for example. The light source supporting member 28 includes a pair of supporting points 28a projecting out on both sides of the light source 26, a pair of legs 28b sticking out below the supporting points 28a, and a pair of release parts 28c sticking out above the supporting points 28a. A claw 28d is formed at the tip of each leg 28b while facing inward. The inner surface of the claw 28d is slanted upward.

When the light source 26 is housed in the light source housing part 14 of the observation container 10, the claw 28d at the tip of each leg 28b of the light source supporting member 28 engages with the supporting step 15. On the other hand, when the release part 28c of the light source supporting member 28 is pinched by fingers, the claw 28d at the tip of each leg 28b is released, so that the light source 26 can be removed from the light source housing part 14 of the observation container 10. The configuration of power supply to the light source 26 will be described later.

The observation container 10 includes a direct-viewing observation container 10a illustrated in FIG. 2 and a side-viewing observation container 10b illustrated in FIG. 3. When the direct-viewing observation container 10a is used, the scope 20 has a direct view at the tissue of the affected part in the patient's body indirectly through the bottom part 11 of the observation container 10a.

As illustrated in FIG. 3, the side-viewing observation container 10b is provided with a reflection part 16 which is arranged below the bottom part 11 of the observation container 10b and tilted to face the bottom part 11. The reflection part 16 is formed of a member having light-reflecting property such as a mirror or a stainless steel plate with mirror finishing that does not transmit light. The reflection part 16 of the present embodiment is set at an angle value of about 90 to 130 degrees upward from a part several millimeters away from the bottom part 11, for example. The angle value however is not limited.

When the side-viewing observation container 10b is used, the scope 20 indirectly observes the affected part in the patient's body from a side through the bottom part 11 and the reflection part 16 of the observation container 10b.

Since an image fiber 22 is formed of a soft elastic synthetic resin, the affectedpart can be observed from various angles by tilting the observation containers 10a and 10b in the body.

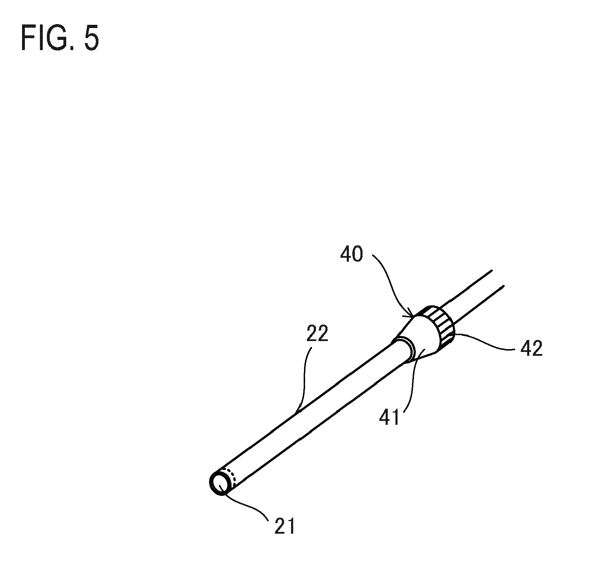

The configuration of insertion and fixation of the scope 20 into the observation container 10 will now be described with reference to FIGS. 1 and 5. FIG. 5 is a schematic perspective view of a mount packing in the present embodiment.

As illustrated in FIGS. 1 and 5, the objective lens 21 is provided at the tip of the scope 20. A cylindrical packing 40 is mounted around the scope 20 at the tip side thereof at which the objective lens 21 is provided, the scope 20 including the image fiberscope and the lens.

The exterior of the packing 40 has a substantially truncated cone shape. That is, an insertion slope 41 is formed at the tip side of the packing 40, and a grip 42 is formed behind the insertion slope 41. The material of the packing 40 may be synthetic rubber such as silicon rubber but is not limited thereto.

The scope 20 can be inserted and fixed in the observation container 10 by inserting the insertion slope 41 of the packing 40 mounted to the scope 20 including the image fiberscope and the lens into a slanted inner diameter part 18 (refer to FIGS. 2A and 3A) of the enlarged-diameter part 13 of the observation container 10.

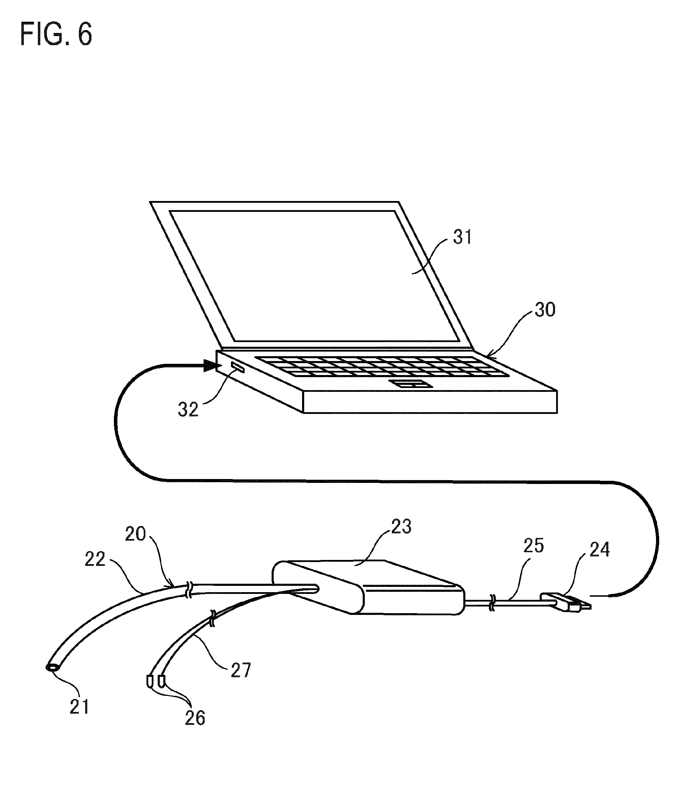

The scope 20, an image processing device 30, and the power supply for the light source 26 will now be described with reference to FIGS. 1 and 6. FIG. 6 is a schematic perspective view of the scope and the image processing device in the present embodiment.

As illustrated in FIGS. 1 and 6, the scope 20 is an instrument which images the condition of the affected part in the patient's body through the bottom part 11 of the observation container 10. As described above, the scope 20 is inserted and fixed into the observation container 10.

The objective lens 21 is provided at the tip of the scope 20. The image fiber 22 of the scope 20 is a light-transmitting member such as an optical fiber and has flexibility.

The fiberscope 20 is connected to a USB cable 25 via a video image signal processing unit 23, the USB cable 25 having at the end thereof a USB connector 24. The video image signal processing unit 23 includes an image output circuit which is not shown.

The video image signal processing unit 23 is electrically connected to a power feed cable 27 for the light source 26 such as the LED lamp. The power for the light source 26 is supplied from a USB terminal 32 of the image processing device (PC) 30 through the USB cable 25 and the video image signal processing unit 23. The LED lamp has small power consumption and is thus suitable for receiving power from the PC. When a number of LED lamps are to be installed, a battery power source may be provided separately since the power from the PC 30 alone would be insufficient.

The image processing device 30 processes information collected from the objective lens 21 of the scope 20 and displays a video image. The personal computer (PC) including a monitor 31 such as a liquid-crystal display panel is used as the image processing device 30. The USB connector 24 of the USB cable 25 is connected to the USB terminal 32 of the PC 30. The image processing device 30 can also have a function to store, reproduce, and analyze an image.

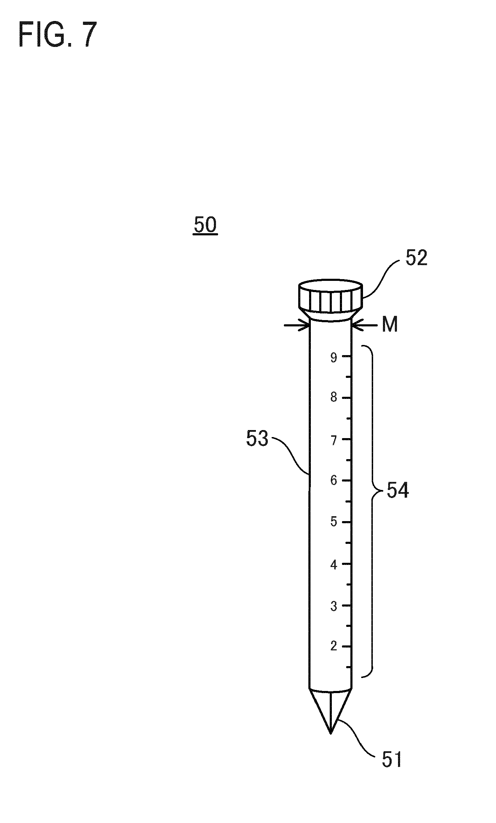

A boring scalpel of the present embodiment will now be described. FIG. 7 is a schematic perspective view of the boring scalpel in the present embodiment.

As illustrated in FIG. 7, the pipe endoscope 100 according to the present embodiment includes a boring scalpel 50 which bores a hole on the body wall such as an abdominal wall in inserting the pipe endoscope 100 into the body. The boring scalpel 50 is a round rod member including at the tip thereof a pointed boring part 51. The material of the boring scalpel 50 can be hard glass, for example, but is not limited thereto.

A grip 52 by which the boring scalpel 50 is gripped is formed at the upper end of the boring scalpel 50. A scale 54 for indicating the boring depth is provided to a body 53 between the boring part 51 and the grip 52. A practitioner can bore a hole to an appropriate depth while referring to the scale 54 provided to the body 53 as a guide.

An outer diameter M of the boring scalpel 50 is set larger than an outer diameter D (refer to FIG. 1) of the observation container 10 since the observation container 10 is inserted into a hole bored by the boring scalpel 50.

### < Operation of pipe endoscope >

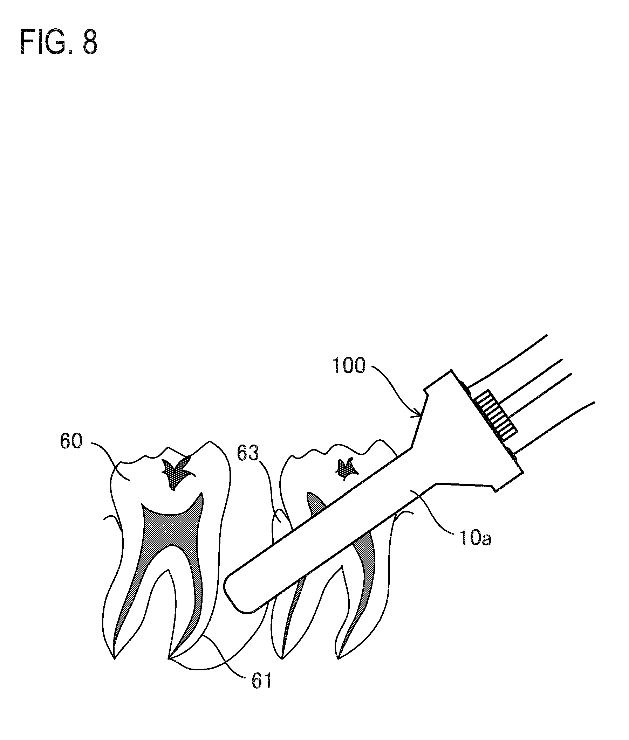

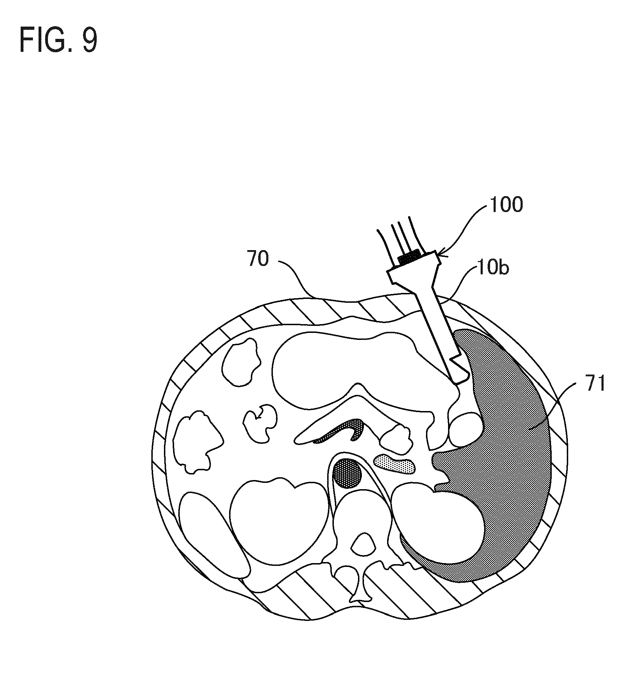

The operation of the pipe endoscope 100 according to the present embodiment will now be described with reference to FIGS. 1 to 9. FIG. 7 is a schematic perspective view of the boring scalpel in the present embodiment. FIG. 8 is a schematic diagram of a use example of the direct-viewing observation container in the pipe endoscope according to the present embodiment. FIG. 9 is a schematic diagram of a use example of the side-viewing observation container in the pipe endoscope according to the present embodiment.

The pipe endoscope 100 according to the present embodiment is used to visually examine the condition of the affected part in the patient's body.

As illustrated in FIG. 5, the packing 40 is first mounted around the tip side of the scope 20 including the image fiberscope and the lens. Next, as illustrated in FIGS. 1 to 3, the tip side of the scope 20 is inserted into the observation container 10. The tip of the scope 20 can be guided into the observation container 10 smoothly because the diameter of the inner diameter part 18 of the enlarged-diameter part 13 in the observation container 10 is gradually increased toward the base end part. The tip side of the scope 20 is inserted and fixed in the observation container 10 by means of the packing 40.

The objective lens 21 is provided at the tip of the scope 20. The objective lens 21 thus faces the bottom part 11 of the observation container 10 when the scope 20 is mounted in the observation container 10.

As illustrated in FIGS. 1 and 4, the light source 26 is set in the light source housing part 14 of the observation container 10, while the claw 28d of the leg 28b of the light source supporting member 28 is supported by the supporting step 15 of the observation container 10.

As illustrated in FIGS. 2 and 3, the observation container 10 of the present embodiment includes the direct-viewing observation container 10a and the side-viewing observation container 10b.

FIG. 8 illustrates a case where a tooth root is diagnosed by visual examination using the direct-viewing observation container 10a of the pipe endoscope 100 according to the present embodiment. Once a patient is administered with an anesthetic, in the diagnosis of the tooth root illustrated in FIG. 8, a gum 63 on the side surface of a tooth root 61 of a tooth 60 to be observed is bored by using the boring scalpel 50 illustrated in FIG. 7 or another scalpel.

When the boring scalpel 50 of the present embodiment is used, the boring depth can easily be checked by referring to the scale 54 provided to the body 53 of the boring scalpel 50 as a guide. Moreover, with the outer diameter M of the boring scalpel 50 being set larger than the outer diameter D of the direct-viewing observation container 10a, a hole larger than the outer diameter D of the observation container 10 can easily be bored (refer to FIGS. 1 and 7).

The tip of the direct-viewing observation container 10a is inserted into the hole bored by the boring scalpel 50 to start the tooth root diagnosis. The objective lens 21 of the scope 20 collects information by imaging the condition of the affected part in the patient's body through the bottom part 11 of the direct-viewing observation container 10a.

The LED lamp as the light source 26 is set in the light source housing part 14 of the direct-viewing observation container 10a (refer to FIG. 1). Light from the LED lamp is guided along the side wall of the direct-viewing observation container 10a and radiated from the bottom part 11. The scope 20 provides enough amount of light to the tooth root 61 for collecting information on the condition of the tooth root 61.

A video image signal from the objective lens 21 is converted in the video image signal processing unit 23 and input to the image processing device 30 via the USB cable 25 (refer to FIG. 6). The image processing device 30 processes a digital video image signal to display the tooth root 61 at a magnification of 5 to 10 times, for example. Themagnified display of the video image allows for the clear visual examination of the tooth root 61, but the magnification is not limited to what is illustrated herein.

FIG. 9 illustrates a case where a tumor in a liver cell is diagnosed by using the side-viewing observation container 10b of the pipe endoscope 100 according to the present embodiment. Once a patient is administered with an anesthetic, in the tumor diagnosis of the liver cell illustrated in FIG. 9, a part of an abdominal wall 70 corresponding to the position of a liver is bored by using the boring scalpel 50 illustrated in FIG. 7 or another scalpel.

When the boring scalpel 50 of the present embodiment is used, as described above, the boring depth can easily be checked as well as the hole larger than the outer diameter D of the observation container 10 can easily be bored (refer to FIGS. 1 and 7).

The tip of the side-viewing observation container 10b is inserted into the hole bored by the boring scalpel 50 to start the tumor diagnosis of the liver cell.

As described above, the scope 20 is mounted in the side-viewing observation container 10b, while the objective lens 21 faces the bottom part 11 of the side-viewing observation container 10b. The reflection part 16 tilted to face the bottom part 11 of the side-viewing observation container 10b is provided at the bottom of the side-viewing observation container 10b. The objective lens 21 of the scope 20 collects information by imaging from the side the tissue of the affected part in the patient's body through the bottom part 11 and the reflection part 16 of the side-viewing observation container 10b.

At this time, the light from the LED lamp is radiated from the bottom part 11 of the side-viewing observation container 10b and reflected by the reflection part 16 (refer to FIGS. 1 and 3). The scope 20 thus provides enough amount of light to the part of a liver 71 to be diagnosed to collect information on the condition of the liver cell.

By tilting the side-viewing observation container 10b, the tissue can be observed from various directions with the scope 20. The condition around the liver 71 can even be observed by rotating the side-viewing observation container 10b in a circumferential direction.

According to the pipe endoscope 100 of the present embodiment, the condition of the affectedpart in the patient' s body can be observed from various directions with the scope 20 by properly using the direct-viewing observation container 10a and the side-viewing observation container 10b for different purposes. At this time, the light from the LED lamp irradiates the affected part in the body so that the scope 20 can collect information on the condition of the affected part by providing enough amount of light to the inside of the body.

The LED lamp is detachably set in the light source housing part 14. The claw 28d at the tip of each leg 28b is released by pinching the release part 28c of the light source supporting member 28 by fingers (refer to FIG. 4), so that the LED lamp can easily be removed from the light source housing part 14. Accordingly, the observation container 10 can solely be made disposable.

The pipe endoscope 100 according to the present embodiment introduces the scope 20 to the affected part in the patient's body through the observation container 10. The scope 20 indirectly collects information on the affected part in the patient's body and thus does not come in direct contact with the environment of the affected part in the body. As a result, the pipe endoscope 100 requiring no sterilization can be realized by having the disposable observation container 10 disposed after each examination. The scope 20 requires no sterilization after each medical treatment, thereby saving both time and a cost of the treatment.

The short-piped protrusion 12 is provided at the tip side of the observation container 10 beyond the bottom part 11 thereof, whereby the bottom part 11 can maintain the superior light-transmitting property without being contaminated with the internal environment of the body. Moreover, the tip of the protrusion 12 has been processed to have the curvature R, thereby making it easier for the endoscope to be inserted into the patient's body and not harming the internal organ or the like of the patient.

Only the observation container 10 and the tip side of the scope 20 of the pipe endoscope 100 according to the present embodiment are inserted into the patient's body, so that the pain felt by the patient can be minimized.

In addition, the pipe endoscope 100 according to the present embodiment includes the LED lamp and has a simple instrument configuration, whereby the reduction in the equipment cost as well as the cost efficiency achieved by the reduction in the examination time due to the simple operation of the instrument can be expected.

While the preferred embodiments of the present invention have been described above for illustrative purposes, the scope of the present invention is not to be limited to these embodiments. The present invention can be implemented in a variety of forms different from the aforementioned embodiments without departing from the spirit of the present invention.

## Claims

1. A pipe endoscope comprising:
a transparent observation container having a bottomed cylindrical shape which is open on a base end side and sealed on a tip side, the observation container including:
a protrusion which is projected to a tip side beyond a sealed bottom part having light transmitting property, and a tip of which is processed to have curvature; and
a detachable light source which irradiates a periphery of the bottom part with light;
a scope which is inserted into the observation container and includes an objective lens at a tip of the scope; and
an image processing device which is connected to the scope and processes information collected by the scope to display a video image.

2. The pipe endoscope according to claim 1, wherein an enlarged-diameter part is formed on an outer wall of a base part of the observation container, the enlarged-diameter part having a stopper function to prevent the entire observation container from entering a patient's body.

3. The pipe endoscope according to claim 1 or 2, wherein an inner diameter part of the enlarged-diameter part includes a slope, a diameter of which is gradually increased toward the base end side of the observation container and which functions as a guide for guiding a tip of the scope.

4. The pipe endoscope according to claim 1 or 2, wherein the protrusion of the observation container includes a reflection part which is tilted upward to face the bottom part of the observation container.

5. The pipe endoscope according to claim 1 or 2, wherein a packing including an insertion slope is mounted around a tip side of the scope, the packing being inserted and fixed into the inner diameter part of the enlarged-diameter part.

6. The pipe endoscope according to claim 1 or 2, comprising a boring scalpel which bores a hole into which the observation container is inserted, the boring scalpel including:
a pointed boring part;
a grip by which the boring scalpel is gripped; and
a body between the boring part and the grip, the body being provided with a scale which indicates a depth of a hole.
